# EUROPÄISCHE PATENTSCHRIFT

(11) **EP 3 386 582 B1**
(45) Veröffentlichungstag und Bekanntmachung des Hinweises auf die Patenterteilung: **06.10.2021**
(21) Anmeldenummer: 16823157.9
(22) Anmeldetag: 08.12.2016
(51) Int. Cl.: A61M 39/24

(54) **SPERR- UND/ODER DROSSELVENTIL UND BLUTBEHANDLUNGSGERÄT UMFASSEND EIN DERARTIGES VENTIL**
SHUT-OFF AND/OR THROTTLING VALVE AND BLOOD TREATMENT DEVICE COMPRISING SUCH A VALVE
CLAPET D'ARRÊT ET/OU D'ÉTRANGLEMENT ET APPAREIL DE TRAITEMENT SANGUIN COMPORTANT UN TEL CLAPET

(30) Priorität: 11.12.2015 DE 102015016023
(43) Veröffentlichungstag der Anmeldung: 17.10.2018
(73) Patentinhaber: Fresenius Medical Care Deutschland GmbH, 61352 Bad Homburg (DE)
(72) Erfinder: OERTER, Gökhan, 35789 Weilmünster (DE)
(74) Vertreter: Herrmann, Uwe
(86) Internationale Anmeldenummer: PCT/EP2016/002067
(87) Internationale Veröffentlichungsnummer: WO 2017/097419

(56) Entgegenhaltungen:
- EP-A1- 1 897 585
- EP-A1- 2 395 269
- WO-A1-2009/029751
- WO-A1-2015/075158
- DE-A1-102008 061 753
- US-A- 4 630 642

## Beschreibung

Die Erfindung betrifft ein Sperr- und/oder Drosselventil und ein Blutbehandlungsgerät mit wenigstens einem derartigen Ventil.

Bei der Dialyse ist die richtige Dosierung von Flüssigkeiten von zentraler Bedeutung. Große Flüssigkeitsströme wie der Dialysierflüssigkeitsstrom werden oft über Bilanziersysteme gefördert. Mittlere Flüssigkeitsströme wie der extrakorporale Blutstrom werden oft durch Schlauchrollenpumpen gefördert. Flussfehler von bis zu etwa 10% werden dabei in Kauf genommen.

Eine höhere Anforderung an die Dosiergenauigkeit besteht aber bei der Zudosierung kleiner Mengen einer Wirkstofflösung, beispielsweise bei der Zudosierung von einer Antikoagulationsmittellösung in den extrakorporalen Blutkreislauf. Hierbei soll der Flussfehler in jedem Fall unter 5% liegen. Im Stand der Technik werden dafür oftmals Schlauchrollenpumpen oder Spritzenpumpen eingesetzt. Der Einsatz von Membranpumpen ist hingegen für derartige Anwendungen im Stand der Technik kaum verbreitet, da unerwünschte Flusspulse erzeugt werden.

Die Flusspulse sind darauf zuückzuführen, dass in realen Membranpumpen die Membran durch ihre Elastizität, durch ihre Verformung und durch ihre Vorspannung das im Idealfall zwischen den beiden Kammern der Membranpumpe herrschende Druckgleichgewicht beeinflusst. Für eine pneumatische Membranpumpe gilt im statischen Fall der Zusammenhang P_{PNEU} - P_{HYD} = P_{MEM}. Der Membrandruck P_{MEM} ist nicht konstant und stellt bei der Regelung der Pumpe einen Störfaktor dar, da sich der Flüssigkeitsdruck P_{HYD} nicht proportional mit dem Regelungsdruck P_{PNEU} verändert. Der Einfluss dieses Störfaktors ist relativ gesehen groß, wenn Flüssigkeitsdruck P_{HYD} und Regelungsdruck P_{PNEU} gering sind. Dies ist beispielsweise dann der Fall, wenn anhand der Membranpumpe geringe Flussraten erzeugt werden sollen.

Vor allem bei der Gabe von Citrat als Antikoagulationsmittel muss aber ein konstanter Fluss aufrechterhalten werden.

Ein der Pumpe nachgelagertes Sperr- und/oder Drosselventil könnte grundsätzlich zum Glätten des Flusses verwendet werden. Bekannte derartige Ventile sind jedoch für derartige Anwendungen lediglich bedingt geeignet, da Gleitreibungseffekte wie ein verzögertes Ansprechen und ein nicht-proportionales Verhalten des Stößels in Reaktion auf eine Ansteuerung (*"Stick-Slip"*-Effekte) auftreten können.

Aus der WO2009/029751A1 ist beispielsweise ein Ventil bekannt, welches den Fluidfluss in einem IV-Set reguliert. Zudem offenbart die Druckschrift EP1897585A1 ein Anti-Siphon- Steuerungsventil. Weiterhin ist aus der US4630642A ein Ventil für einen Turbolader bekannt. Die Druckschrift WO2015/075158A1 offenbart ein Ventil für einen medizinischen Ventilator.

Ziel der Erfindung ist es vor diesem Hintergrund, ein Sperrventil bereitzustellen, in dem keine Gleitreibungseffekte auftreten und das proportional regelbar ist.

Vor diesem Hintergrund betrifft die Erfindung, die im ersten Hauptanspruch definiert ist, ein Sperr- und/oder Drosselventil mit einem Ventilgehäuse, das ein Fluidsystem mit einem Fluideinlass, einem Fluidauslass und einer dazwischen angeordneten Fluidkammer aufweist. Die Fluidkammer umfasst eine Sperrgeometrie und wird an einer der Sperrgeometrie gegenüberliegenden Seite von einer Sperrmembran begrenzt. Erfindungsgemäß zeichnet sich das Ventil dadurch aus, dass die Sperrmembran auf ihrer der Fluidkammer abgewandten Seite derart mit einem Stößel in Verbindung steht, dass sie durch eine Bewegung des Stößels an die Absperrgeometrie gedrückt und von dieser gehoben werden kann. Dabei ist erfindungsgemäß vorgesehen, dass der Stößel frei schwebend in einem Hohlraum des Ventilgehäuses angeordnet ist.

Unter einer frei schwebenden Lagerung des Stößels ist eine reibungsfreie Lagerung zu verstehen, in der die Oberfläche des Stößels bei einer Bewegung des Stößels nicht an einem stationären Element des Ventilgehäuses wie beispielsweise einer Dichtung entlanggleitet und sich daran reibt.

In einer Ausführungsform ist zwischen dem Stößel und dem Ventilgehäuse eine mechanische Feder angeordnet, die das Lager des frei schwebenden Stößels bilden kann und den Stößel so gegen das Ventilgehäuse vorspannt, dass er die Sperrmembran in seiner Nullstellung an die Absperrgeometrie drückt. Ferner kann das Ventil einen auf den Stößel wirkenden Aktor aufweisen, der so ausgebildet ist, dass er den Stößel gegen die Vorspannung der mechanischen Feder aus der Nullstellung bewegen und so die Sperrmembran von der Absperrgeometrie lösen kann.

In einer alternativen Ausführungsform ist zwischen dem Stößel und dem Ventilgehäuse eine mechanische Feder angeordnet, die das Lager des frei schwebenden Stößels bilden kann und den Stößel so gegen das Ventilgehäuse vorspannt, dass er die Sperrmembran in seiner Nullstellung von der Absperrgeometrie löst. Ferner kann das Ventil einen auf den Stößel wirkenden Aktor aufweisen, der so ausgebildet ist, dass er den Stößel gegen die Vorspannung der mechanischen Feder aus der Nullstellung bewegen und so die Sperrmembran an die Absperrgeometrie drücken kann.

In einer Ausführungsform handelt es sich bei der mechanischen Feder um eine als Druckfeder wirkende und beispielsweise metallische oder keramische Schraubenfeder.

In einer Ausführungsform handelt es sich bei dem Aktor um einen pneumatischen Aktor. Alternativ kann auch ein mechanischer oder hydraulischer Aktor vorgesehen sein.

In einer Ausführungsform umfasst der pneumatische Aktor eine Aktormembran, die an eine Blähkammer grenzt oder eine Blähkammer umschließt. Die Blähkammer weist eine Schnittstelle zum Anschluss einer pneumatischen Druckleitung auf. Die Aktormembran ist so angeordnet und konfiguriert, dass sie im aktiven Zustand des Aktors gegen den Stößel drücken und so auf diesen wirken kann.

In einer Ausführungsform ist der Stößel stiftförmig und weist vorzugsweise einen zwischen seinen beiden gegenüberliegenden Enden angeordneten radialen Flansch auf.

In einer Ausführungsform ist der Stößel an einem Ende mit der Sperrmembran und am anderen Ende mit der Aktormembran verbunden. Ferner ist in dieser Ausführungsform vorgesehen, dass die mechanische Feder an der der Sperrmembran zugewandten Seite des Flansches an den Stößel angreift.

In einer alternativen Ausführungsform ist der Stößel an einem Ende mit der Sperrmembran verbunden. Ferner ist in dieser Ausführungsform vorgesehen, dass die mechanische Feder an der der Sperrmembran abgewandten Seite des Flansches an den Stößel angreift und dass die der Sperrmembran zugewandte Seite des Flansches eine Auflagefläche für die Aktormembran bildet.

In einer Ausführungsform umfasst das Ventilgehäuse zwei lösbar aneinander befestigte Teile, wobei ein erster Teil den Hohlraum für den Stößel und ein zweiter Teil die Fluidkammer definiert, und wobei die Sperrmembran zwischen den beiden Teilen angeordnet ist. Die Sperrmembran kann dabei in einer Ausführungsform ebenfalls von dem ersten Teil abgenommen werden. Bei dem ersten Teil handelt es sich in dieser Ausführungsform um ein Reusable, welches nicht mit dem Fluidweg in Kontakt steht und so vor Kontamination durch das Fluid geschützt ist. Nur die austauschbare Sperrmembran und der zweite Teil, bei dem es sich um ein austauschbares Reusable handelt, kommen mit dem Fluid in Kontakt.

Bei der Sperrmembran und/oder der Aktormembran kann es sich beispielsweise um eine runde Silikonscheibe oder eine Silikonplatte handeln.

Bei dem Ventil kann es sich um ein Zwei-Wege-Sperr- und/oder Drosselventil handeln, das genau einen Fluideinlass und genau einen Fluidauslass aufweist.

Vor dem eingangs genannten Hintergrund betrifft die Erfindung ferner ein Blutbehandlungsgerät mit wenigstens einem erfindungsgemäßen Sperr- und/oder Drosselventil. Bei dem Blutbehandlungsgerät handelt es sich vorzugsweise um ein Dialysegerät.

In einer Ausführungsform ist das Ventil in einer Dosierleitung angeordnet, die in den Dialysierflüssigkeitskreislauf oder in den extrakorporalen Blutkreislauf mündet. Vorzugsweise ist das Ventil in einer Dosierleitung für eine Lösung eines koagulationshemmenden Mittels wie beispielsweise Heparin oder Citrat angeordnet, die in den extrakorporalen Blutkreislauf mündet.

Weitere Einzelheiten und Vorteile der Erfindung ergeben sich aus den anhand der Figuren beschriebenen Ausführungsbeispielen. In den Figuren zeigen:
- Figur 1:: eine Ausführungsform eines erfindungsgemäßen Normally-Opened-Sperr- und/oder Drosselventils; und
- Figur 2:: eine Ausführungsform eines erfindungsgemäßen Normally-Closed-Sperr- und/oder Drosselventils.

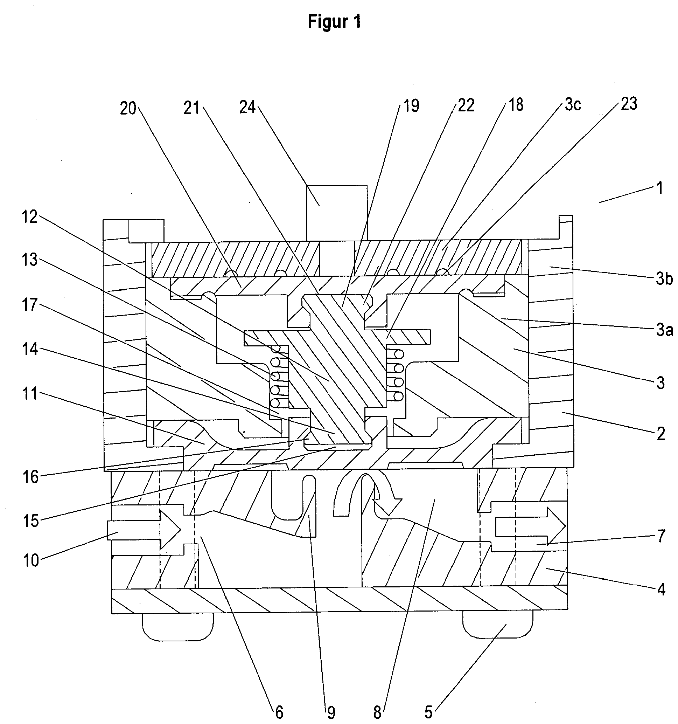

Figur 1 zeigt ein erfindungsgemäßes Sperr- und/oder Drosselventil, welches allgemein mit dem Bezugszeichen 1 gekennzeichnet ist.

Das Ventil weist ein Ventilgehäuse 2 auf, das einen wiederverwendbaren Teil ("*Reusable*") 3 und einen lösbar daran befestigten Einwegteil ("*Disposable*") 4 umfasst. Das Disposable 4 ist anhand von lösbaren Bolzen 5 am Reusable 3 befestigt. Das Reusable 3 umfasst seinerseits einen Einsatz 3a, der in eine Ausnehmung einer Maschinenplatte 3b eingelegt ist und von einer Abschlussplatte 3c bedeckt wird.

Das Disposable 4 umfasst einen Fluidweg mit einem Fluideinlass 6, einem Fluidauslass 7 und einer dazwischen angeordneten Fluidkammer 8. Innerhalb der Fluidkammer ist eine Sperrgeometrie 9 ausgebildet, bei welcher es sich um einen die runde Kammereinlassöffnung kragenartig umlaufenden und in die Fluidkammer 8 ragenden Steg handelt. Der Fluidfluss ist mit den Pfeilen 10 erkennbar gemacht.

An der der Sperrgeometrie 9 gegenüberliegenden Seite wird die Fluidkammer 8 durch eine Sperrmembran 11 in Form einer Silikonplatte begrenzt. Die Silikonplatte ist überdeckt eine konzentrlisch zur runden Kammereinlassöffnung angeordnete runde Öffnung in der Wand der Fluidkammer 8.

Die Silikonplatte 11 ist lösbar zwischen dem Einsatz 3a und der Maschinenplatte in das Reusable 3 eingelegt und schließt auf allen Seiten der Fluidkammer 8 bündig mit dem Disposable 4, sodass kein Abschnitt des Reusables 3 mit dem Fluidweg in Kontakt steht. Auf diese Weise ist das Reusable 3 vor Kontamination durch das Fluid geschützt und nur die austauschbare Silikonplatte 11 und das austauschbare Reusable 4 kommen mit dem Fluid in Kontakt.

Hinter der Silikonplatte 11 ist im Reusable 3 ein Hohlraum ausgebildet, in der ein stiftförmiger Stößel 12 zur Auslenkung der Silikonplatte 11 angeordnet ist. Der Stößel umfasst einen zwischen seinem der Fluidkammer zugewandten Endbereich 14 (der "*Sperrseite*") und seinem der Fluidkammer abgewandten Endbereich 19 (der *"Aktorseite*") einen radial umlaufenden Flansch 18. Die Zentralachse des Stößels 12 verläuft durch den Mittelpunkt der Kammereinlassöffnung. Der Stößel 12 kann innerhalb des Hohlraums linear entlang der Zentralachse hin und her bewegt und insoweit auf die Fluidkammer zubewegt und von dieser wegbewegt werden. Er wird von einer beispielsweise metallischen Schraubenfeder 13 frei schwebend zwischen den Wänden des Hohlraums gehalten, wobei Achsen des Stößels 12 und der Schraubenfeder 13 konzentrisch zueinander sind. Die Schraubenfeder 13 greift den Stößel 12 an der der Fluidkammer zugewandten Seite des Flansches 18 an und wirkt dabei als Druckfeder. Am anderen Ende liegt die Schraubenfeder auf einem Vorsprung 17 im Disposable 3 auf. Ein fluidkammerseitig des Flansches 18 angeordneter Abschnitt des stiftförmigen Stößelkörpers verläuft innerhalb der Schraubenfeder 13.

Die Sperrseite 14 des Stößels 12 umfasst einen Halteflansch 16 und an der Silikonplatte 11 ist hohlraumseitig eine Aufnahme 15 für die Sperrseite 14 des Stößels 12 angeformt, die eine zum Halteflansch 16 korrespondierende Hinterschneidung aufweist. Auf diese Weise können sowohl Zug- als auch Druckkräfte vom Stößel 12 auf die Silikonplatte 11 im mit der Sperrgeometrie 9 fluchtenden Bereich übertragen werden. Anhand dieser Anordnung wird einerseits erreicht, dass die Silikonplatte 11 bei einer in Richtung der Fluidkammer gerichteten (Schließ-)Bewegung des Stößels 12 in Richtung der Sperrgeometrie 9 gedrückt wird, was im Betrieb des Ventils 1 ein Drosseln oder Sperren des Fluidflusses bewirkt, und andererseits, dass die Silikonplatte 11 bei einer von der Fluidkammer weggerichteten (Öffnungs-)Bewegung des Stößels 12 von der Sperrgeometrie 9 gehoben wird, was im Betrieb des Ventils 1 ein Öffnen oder Vergrößern des Fluidflusses bewirkt.

Eine zwischen der Abdeckplatte 3c und dem Einsatz 3a geklemmte Aktormembran 20 in Form einer runden Silikonscheibe begrenzt den Hohlraum an seinem der Silikonplatte 11 gegenüberliegenden Ende. Die Silikonscheibe 20 ist parallel zur Silikonplatte 11 angeordnet und der Mittelpunkt der Silikonscheibe 20 liegt in der Achse des Stößels 12.

Auch die Silikonscheibe 20 steht mit dem Stößel 12 in Verbindung, genauer mit der Aktorseite 19 des Stößels. Zu diesem Zweck umfasst auch die Aktorseite 19 des Stößels 12 umfasst einen Halteflansch 22 und auch an der Silikonscheibe 20 ist hohlraumseitig eine Aufnahme 21 angeformt, die eine zum Halteflansch 22 korrespondierende Hinterschneidung aufweist. So können Druckkräfte von der Silikonscheibe 20 auf den Stößel 12 übertragen werden und der Stößel kann im Bereich des Mittelpunktes der Silikonscheibe 20 gehalten werden. Anhand dieser Anordnung wird erreicht, dass der Stößel 12 bei einer in den Hohlraum drückenden Verformung der Silikonscheibe 20 eine gegen die Druckkraft der Feder 13 und in Richtung der Fluidkammer gerichtete (Schließ-)Bewegung vollzieht. Eine

Zwischen der Silikonscheibe 20 und der Abdeckplatte ist eine Blähkammer 23 ausgebildet. Bei Erzeugung eines Fluiddrucks in der Blähkammer 23 erfolgt eine ballonförmige Ausdehnung (und so eine in den Hohlraum drückende Verformung) der Silikonscheibe 20. Zum Anschluss eines beispielsweise pneumatischen Steuersystems ist in der Abschlussplatte 3c ein Anschluss 24 eingearbeitet. Der pneumatische Anschluss liegt wiederum in der Achse des Stößels 12.

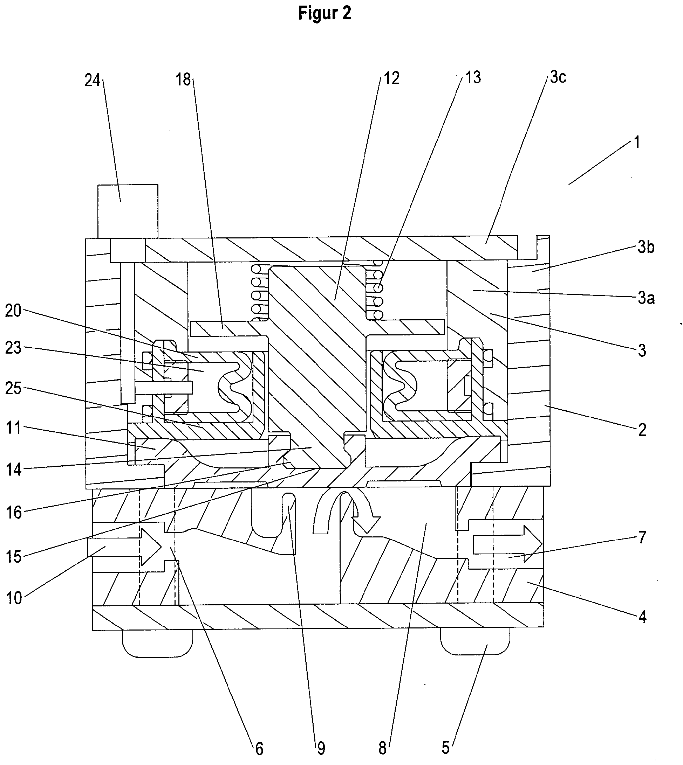

Wird in der Blähkammer 23 ein Überdruck erzeugt, so drückt die Silikonscheibe 20 auf den Stößel 12 und bewegt diesen gegen die von der Feder 13 ausgehende Druckkraft in Richtung der Fluidkammer 8, wodurch die Silikonplatte 11 in Richtung der Sperrgeometrie 9 gedrückt wird, was ein Drosseln oder Schließen des Fluidwegs bewirkt. Lässt der Überdruck nach, so drückt die Feder 13 den Stößel 12 gegen die Blähkammer 23, wodurch die Silikonplatte 11 von der Sperrgeometrie 9 gehoben wird, was ein Öffnen oder Vergrößern des Fluidwegs bewirkt. Da die Feder 13 den Stößel 12 von der Fluidkammer 8 und so die Silikonplatte 11 von der Sperrgeometrie 9 hebt, ist das in Figur 2 gezeigte Ventil in seiner Nullstellung (bei einem Ausfall des Steuersystems, ggf. *"stromlos*") geöffnet.

Figur 2 zeigt eine weitere Ausführungsform eines erfindungsgemäßes Sperr- und/oder Drosselventils, wobei identische bzw. funktionsäquivalente Teile mit identischen Bezugszeichen gekennzeichnet sind, wie dies in Figur 1 der Fall ist.

Der zentrale Unterschied gegenüber dem in Figur 1 gezeigten Ventil besteht darin, dass das in Figur 2 gezeigte Sperr- und/oder Drosselventil in seiner Nullstellung (bei einem Ausfall des Steuersystems, ggf. *"stromlos*") geschlossen ist.

Was den konstruktiven Aufbau betrifft, unterscheidet sich das Normally-Closed-Ventil gemäß Figur 2 von dem Normally-Open-Ventil der Figur 1 dadurch, dass die mechanische Druckfeder 13 nicht an der fluidkammerseitigen Seite des Flansches 18 auf den Stößel wirkt, sondern an der fluidkammerabgewandten Seite. Die Basis der Druckfeder 13 stützt sich daher in der Ausführungsform gemäß Figur 2 nicht gegen einem Vorsprung 17 im Hohlraum des Disposables 3 sondern gegen die Abdeckplatte 3c. Demgegenüber wirkt der pneumatische Aktor bestehend aus Blähkammer 23 und Aktormembran 20 vorliegend an der fluidkammerseitigen Seite des Flansches 18 statt an der Aktorseite 19. Die spezielle Ausbildung der Aktorseite mit einem Halteflansch 22 kann daher entfallen.

Der pneumatische Aktor umfasst vorliegend keine Silikonscheibe, wie dies in der Ausführungsform gemäß Figur 1 der Fall ist, sondern einen blähbaren Schlauch 20, der den Stößel 12 radial umschließt und fluidkammerseiteig des Flansches 18 im Hohlraum angeordnet ist. Zur Aufnahme dieses Schlauchs 20 ist im Hohlraum in dem Bereich, in dem in der Ausführungsform gemäß Figur 1 der Vorsprung 17 zur Abstützung der Feder 13 angeordnet ist, eine ringförmige Aufnahme 25 vorgesehen, die in Richtung des Flansches offen ist.

Wird in der vom Schlauch 20 umschlossenen Blähkammer 23 ein Überdruck erzeugt, so bläht sich der Schlauch auf und drückt gegen den Flansch des Stößels, sodass dieser gegen die von der Feder 13 ausgehende Druckkraft in Richtung der Abschlussplatte 3c, wodurch die Silikonplatte 11 von der Sperrgeometrie 9 gehoben wird, was ein Öffnen oder Vergrößern Fluidwegs bewirkt. Lässt der Überdruck nach, so drückt die Feder 13 den Stößel 12 wiederum in Richtung der Fluidkammer 8, was ein Drosseln oder Schließen des Fluidwegs bewirkt. Da die Feder 13 den Stößel 12 in Richtung der Fluidkammer 8 und so die Silikonplatte 11 an die Sperrgeometrie 9 drückt, ist das in Figur 2 gezeigte Ventil in seiner Nullstellung (bei einem Ausfall des Steuersystems, ggf. *"stromlos*") geschlossen.

Der Anschluss 24 für ein beispielsweise pneumatisches Steuersystem an die Blähkammer 23 greift radial an den Schlauch 20 an.

Figur 2 zeigt das Ventil 1 nicht in seiner Nullstellung sondern aus illustrativen Zwecken in einer (während des Betriebs nicht auftretenden) Stellung, in der der Stößel 12 trotz fehlender Aufblähung des Schlauchs 20 aus seiner Nullstellung gehoben ist.

Ein zentraler Aspekt der Erfindung liegt mit Blick auf beide Ausführungsformen darin, dass der Stößel 12 innerhalb des Hohlraums frei schwebend angeordnet ist und die Oberfläche des Stößels 12 nicht an einer Dichtlippe wie beispielsweise einem Dichtring anliegt. So werden Gleitreibungseffekte wie ein verzögertes Ansprechen und ein nicht-proportionales Verhalten des Stößels in Reaktion auf eine Druckerhöhung ("*Stick-Slip"*-Effekte) vermieden, die insbesondere bei einer pneumatischen Ansteuerung mit einem kompressiblen Gas ansonsten stark ausgeprägt sein können. Das erfindungsgemäße Ventil 1 ist pneumatisch streng proportional regelbar. Durch die Proportionalität kann nicht nur zwischen zwei Zuständen (geschlossen, geöffnet) unterschieden werden, sondern das Ventil kann auch die Funktion einer Drossel übernehmen.

## Patentansprüche

1. Sperr- und/oder Drosselventil (1) eines Blutbehandlungsgeräts, mit einem Ventilgehäuse (2), das ein Fluidsystem mit einem Fluideinlass (6), einem Fluidauslass (7) und einer dazwischen angeordneten Fluidkammer (8) aufweist, wobei die Fluidkammer (8) eine Sperrgeometrie (9) umfasst und gegenüber der Sperrgeometrie (9) von einer Sperrmembran (11) begrenzt wird, wobei die Sperrmembran (11) auf ihrer der Fluidkammer (8) abgewandten Seite derart mit einem Stößel (12) in Verbindung steht, dass sie durch eine Bewegung des Stößels (12) an die Absperrgeometrie (9) gedrückt und von dieser gehoben werden kann, **dadurch gekennzeichnet, dass** der Stößel (12) derart in einem Hohlraum des Ventilgehäuses (2) angeordnet ist, dass die Oberfläche des Stößels (12) bei einer Bewegung des Stößels (12) nicht an einem stationären Element des Ventilgehäuses (2) entlanggleitet und sich daran reibt, und dass zwischen Stößel (12) und Ventilgehäuse (2) eine mechanische Feder (13) angeordnet ist, die den Stößel (12) so gegen das Ventilgehäuse (2) vorspannt, dass er die Sperrmembran (11) in seiner Nullstellung an die Absperrgeometrie (9) drückt oder von dieser löst.

2. Sperr- und/oder Drosselventil nach Anspruch 1, **dadurch gekennzeichnet, dass** es ferner einen auf den Stößel (12) wirkenden pneumatischen Aktor aufweist, der so ausgebildet ist, dass er den Stößel (12) aus gegen die Vorspannung der mechanischen Feder (13) aus der Nullstellung bewegen und so die Sperrmembran (11) von der Absperrgeometrie (9) lösen oder an diese drücken kann.

3. Sperr- und/oder Drosselventil nach Anspruch 2, **dadurch gekennzeichnet, dass** der pneumatische Aktor eine Aktormembran (20) umfasst, die an eine Blähkammer (23) grenzt oder eine Blähkammer (23) umschließt, wobei die Blähkammer (23) eine Schnittstelle (24) zum Anschluss einer pneumatischen Druckleitung aufweist.

4. Sperr- und/oder Drosselventil nach einem der vorhergehenden Ansprüche, **dadurch gekennzeichnet, dass** der Stößel (12) stiftförmig ist und vorzugsweise einen zwischen seinen beiden gegenüberliegenden Enden (14, 19) angeordneten radialen Flansch (18) aufweist.

5. Sperr- und/oder Drosselventil nach Anspruch 4, **dadurch gekennzeichnet, dass** der Stößel (12) an einem Ende (14) mit der Sperrmembran (11) und am anderen Ende mit der Aktormembran (20) verbunden ist, wobei ferner vorgesehen ist, dass die mechanische Feder (13) an der der Sperrmembran (11) zugewandten Seite des Flansches (18) an den Stößel (12) angreift.

6. Sperr- und/oder Drosselventil nach Anspruch 4, **dadurch gekennzeichnet, dass** der Stößel (12) an einem Ende (14) mit der Sperrmembran (11) verbunden ist, wobei ferner vorgesehen ist, dass die mechanische Feder (13) an der der Sperrmembran (11) abgewandten Seite des Flansches (18) an den Stößel (12) angreift und die der Sperrmembran (11) zugewandte Seite des Flansches (18) eine Auflagefläche für die Aktormembran (20) bildet.

7. Sperr- und/oder Drosselventil nach einem der vorhergehenden Ansprüche, **dadurch gekennzeichnet, dass** das Ventilgehäuse (2) zwei lösbar aneinander befestigte Teile (3, 4) umfasst, wobei ein erster Teil (3) den Hohlraum für den Stößel (12) und ein zweiter Teil (4) die Fluidkammer definiert, und wobei die Sperrmembran (11) zwischen den beiden Teilen (3, 4) angeordnet ist.

8. Blutbehandlungsgerät mit wenigstens einem Sperr- und/oder Drosselventil nach einem der vorhergehenden Ansprüche, wobei es sich bei dem Blutbehandlungsgerät vorzugsweise um ein Dialysegerät handelt.

9. Blutbehandlungsgerät nach Anspruch 8, **dadurch gekennzeichnet, dass** das Sperr- und/oder Drosselventil in einer Dosierleitung angeordnet ist, die in den Dialysierflüssigkeitskreislauf oder in den extrakorporalen Blutkreislauf mündet, wobei das Sperr- und/oder Drosselventil vorzugsweise in einer Dosierleitung für eine Lösung eines koagulationshemmenden Mittels angeordnet ist, die in den extrakorporalen Blutkreislauf mündet.

## Claims

1. A blocking and/or restricting valve (1) of a blood treatment device having a valve housing (2) which has a fluid system having a fluid inlet (6), a fluid outlet (7) and a fluid chamber (8) arranged therebetween, wherein the fluid chamber (8) comprises a blocking geometry (9) and is bounded by a barrier membrane (11) with respect to the blocking geometry (9), wherein the barrier membrane (11) is connected to a tappet (12) on its side remote from the fluid chamber (8) such that it is pressed toward the blocking geometry (9) and can be lifted by it by a movement of the tappet (12), **characterized in that** the tappet (12) is arranged in a hollow space of the valve housing (2) such that the surface of the tappet (12) does not slide along a stationary element of the valve housing (2) and does not rub on it on a movement of the tappet; and **in that**
a mechanical spring (13) is arranged between the tappet (12) and the valve housing (2), said spring pre-loading the tappet (12) against the valve housing (2) such that it presses the barrier membrane (11) toward the blocking geometry (9) or releases it therefrom in its zero position.

2. A blocking and/or restricting valve in accordance with claim 1, **characterized in that** it furthermore has a pneumatic actuator which acts on the tappet (12) and which is configured such that it can move the tappet (12) out of the zero position against the pre-load of the mechanical spring (13) and can thus release the barrier membrane (11) from the blocking geometry (9) or can press it toward it.

3. A blocking and/or restricting valve in accordance with claim 2, **characterized in that** the pneumatic actuator comprises an actuator membrane (20) which is adjacent to an inflation chamber (23) or which surrounds an inflation chamber (23), with the inflation chamber (23) having an interface (24) for the connection of a pneumatic pressure line.

4. A blocking and/or restricting valve in accordance with one of the preceding claims, **characterized in that** the tappet (12) is of pin shape and preferably has a radial flange (18) arranged between its two oppositely disposed ends (14, 19).

5. A blocking and/or restricting valve in accordance with claim 4, **characterized in that** the tappet (12) is connected to the barrier membrane (11) at one end (14) and is connected to the actuator membrane (20) at the other end, with provision furthermore being made that the mechanical spring (13) engages at the tappet (12) at the side of the flange (18) facing the barrier membrane (11).

6. A blocking and/or restricting valve in accordance with claim 4, **characterized in that** the tappet (12) is connected to the barrier membrane (11) at one end (14), with provision furthermore being made that the mechanical spring (13) engages at the tappet (12) at the side of the flange (18) remote from the barrier membrane (11) and the side of the flange (18) facing the barrier membrane (11) forms a support surface for the actuator membrane (20).

7. A blocking and/or restricting valve in accordance with one of the preceding claims, **characterized in that** the valve housing (2) comprises two parts (3, 4) which are releasably fastened to one another, with a first part (3) defining the hollow space for the tappet (12) and with a second part (4) defining the fluid chamber, and with the barrier membrane (11) being arranged between the two parts (3, 4).

8. A blood treatment device having at least one blocking and/or restricting valve in accordance with one of the preceding claims, wherein the blood treatment device is preferably a dialysis device.

9. A blood treatment device in accordance with claim 8, **characterized in that** the blocking and/or restricting valve is arranged in a metering line which opens into the dialysis fluid circuit or into the extracorporeal blood circuit, with the blocking and/or restricting valve preferably being arranged in a metering line for a solution of a coagulation-inhibiting agent which opens into the extracorporeal blood circuit.

## Revendications

1. Clapet d'arrêt et/ou d'étranglement (1) d'un appareil de traitement sanguin, comprenant un boîtier de clapet (2), qui comporte un système de fluide avec une entrée de fluide (6), une sortie de fluide (7) et une chambre de fluide (8) disposée entre celles-ci, la chambre de fluide (8) comprenant une géométrie d'arrêt (9) et étant limitée, à l'opposé de la géométrie d'arrêt (9), par une membrane d'arrêt (11), la membrane d'arrêt (11) étant reliée, sur son côté orienté dans la direction opposée à la chambre de fluide (8), à une tige-poussoir (12) de telle manière qu'elle peut être appuyée, par un mouvement de la tige-poussoir (12), sur la géométrie d'arrêt (9) et soulevée de celle-ci,
**caractérisé en ce que** la tige-poussoir (12) est disposée dans une cavité du boîtier de clapet (2) de telle manière que la surface de la tige-poussoir (12), lors d'un mouvement de la tige-poussoir (12), ne glisse pas le long d'un élément fixe du boîtier de clapet (2) et ne s'y frotte pas, et **en ce que**, entre la tige-poussoir (12) et le boîtier de clapet (2), se trouve un ressort mécanique (13), qui précontraint la tige-poussoir (12) contre le boîtier de clapet (2) de telle sorte qu'elle appuie la membrane d'arrêt (11) dans sa position neutre sur la géométrie d'arrêt (9) ou l'en sépare.

2. Clapet d'arrêt et/ou d'étranglement selon la revendication 1, **caractérisé en ce qu'**il comporte en outre un actionneur pneumatique agissant sur la tige-poussoir (12), qui est conçu de telle manière qu'il peut déplacer la tige-poussoir (12) hors de la position neutre à l'encontre de la précontrainte du ressort mécanique (13) et ainsi séparer la membrane d'arrêt (11) de la géométrie d'arrêt (9) ou l'appuyer contre celle-ci.

3. Clapet d'arrêt et/ou d'étranglement selon la revendication 2, **caractérisé en ce que** l'actionneur pneumatique comprend une membrane d'actionneur (20), qui est contiguë à une chambre de gonflement (23) ou entoure une chambre de gonflement (23), la chambre de gonflement (23) comportant une interface (24) pour le raccordement d'une ligne sous pression pneumatique.

4. Clapet d'arrêt et/ou d'étranglement selon l'une des revendications précédentes, **caractérisé en ce que** la tige-poussoir (12) est en forme de tige et comporte de préférence un collet (18) radial disposé entre ses deux extrémités (14, 19) opposées.

5. Clapet d'arrêt et/ou d'étranglement selon la revendication 4, **caractérisé en ce que** la tige-poussoir (12) est reliée par une extrémité (14) à la membrane d'arrêt (11) et par l'autre extrémité à la membrane d'actionneur (20), dans lequel il est en outre prévu que le ressort mécanique (13) saisisse la tige-poussoir (12) sur le côté du collet (18) orienté vers la membrane d'arrêt (11).

6. Clapet d'arrêt et/ou d'étranglement selon la revendication 4, **caractérisé en ce que** la tige-poussoir (12) est reliée, par une extrémité (14), à la membrane d'arrêt (11), dans lequel il est en outre prévu que le ressort mécanique (13) saisisse la tige-poussoir (12) sur le côté du collet (18) orienté dans la direction opposée à la membrane d'arrêt (11) et que le côté du collet (18) orienté vers la membrane d'arrêt (11) forme une surface d'appui pour la membrane d'actionneur (20).

7. Clapet d'arrêt et/ou d'étranglement selon l'une des revendications précédentes, **caractérisé en ce que** le boîtier de clapet (2) comprend deux parties (3, 4) fixées l'une à l'autre de manière détachable, une première partie (3) définissant la cavité pour la tige-poussoir (2) et une seconde partie (4) la chambre de fluide, et la membrane d'arrêt (11) étant disposée entre les deux parties (3, 4).

8. Appareil de traitement sanguin comprenant au moins un clapet d'arrêt et/ou d'étranglement selon l'une des revendications précédentes, l'appareil de traitement sanguin étant de préférence un appareil de dialyse.

9. Appareil de traitement sanguin selon la revendication 8, **caractérisé en ce que** le clapet d'arrêt et/ou d'étranglement est disposé dans une ligne de dosage, qui débouche dans le circuit de fluide de dialyse ou dans le circuit sanguin extracorporel, le clapet d'arrêt et/ou d'étranglement étant de préférence disposé dans une ligne de dosage pour une solution d'un anticoagulant, qui débouche dans le circuit sanguin extracorporel.
